(19) Europäisches Patentamt
European Patent Office
Office européen des brevets

(11) Veröffentlichungsnummer : **0 133 285 B1**

(12) # EUROPÄISCHE PATENTSCHRIFT

(45) Veröffentlichungstag der Patentschrift : **02.03.88**

(51) Int. Cl.⁴ : **C 07 K 7/40**, C 07 K 7/42, A 61 K 37/26

(21) Anmeldenummer : **84108769.5**

(22) Anmeldetag : **25.07.84**

(54) **Insulin-Derivat-Kristallsuspensionen, Verfahren zu deren Herstellung und deren Verwendung.**

(30) Priorität : **29.07.83 DE 3327709**

(43) Veröffentlichungstag der Anmeldung : **20.02.85 Patentblatt 85/08**

(45) Bekanntmachung des Hinweises auf die Patenterteilung : **02.03.88 Patentblatt 88/09**

(84) Benannte Vertragsstaaten : **AT BE CH DE FR GB IT LI LU NL SE**

(56) Entgegenhaltungen :
**EP-A- 0 045 187**
**EP-A- 0 092 280**
**DE-A- 2 018 588**
**GB-A- 729 670**
**J. SCHLICHTKRULL "Insulincrystals", Copenhagen, Ejnar Munksgaard, Publishers, 1958, Seiten 51-56**
**Die Akte enthält technische Angaben, die nach dem Eingang der Anmeldung eingereicht wurden und die nicht in dieser Patentschrift enthalten sind.**

(73) Patentinhaber : **HOECHST AKTIENGESELLSCHAFT Postfach 80 03 20 D-6230 Frankfurt am Main 80 (DE)**

(72) Erfinder : **Grau, Ulrich, Dr. Zeil 17 D-6238 Hofheim am Taunus (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 133 285 B1

Jouve, 18, rue St-Denis, 75001 Paris, France

**Beschreibung**

Die Kristallisation von Insulin ist sowohl im Hinblick die strukturanalystischen Arbeiten (Adams et al., Nature 224, 491 (1969), The Peking Insulin Structure Research Group, Sc. Sinica XVII, 152-118 (1974)) als auch im Hinblick auf pharmazeutische Anwendungen (Schlichtkrull, Insulin-Kristalle (1961)), ein intensiv bearbeitetes Gebiet. Für die therapeutische Anwendung als Verzögerungsform des Insulins steht dabei im Vordergrund eine möglichst definierte Kristallgröße, die üblicherweise ca. 30 µm nicht überschreiten sollte, so daß die Oberfläche in direkter und reproduzierbarer Weise mit der Absolutmenge an Insulin zusammenhängt. Mit solchen sog. monodispersen Suspensionen ist eine definierte Wiederauflösungskinetik zu erwarten.

Beispiele für therapeutisch angewandte Insulinkristallsuspensionen mit solchen Eigenschaften sind Suspensionen aus ca. 25 µm großen rhomboedrischen Zink-Insulinkristallen, die in Gegenwart von 0,8 bis 2,5 % Zink (bezogen auf die Insulinmasse) bei neutralem pH-Wert stabil sind und eine verzögerte Wirkung zeigen, sowie Isophan-Insulin-Protamin-Kristalle, die in Form von ca. 10 µm langen und ca. 1 µm dicken Stäbchen in Verzögerungspräparaten Anwendung finden.

Darüberhinaus sind einige weitere Kristallmodifikationen des Insulins bekannt, die aber bisher nur für die Röntgenstrukturanalyse von Interesse waren. So wurden unter sauren pH-Bedingungen Zink-freie orthorhombische und monokline Kristalle erhalten (Einstein, Low, Acta Cryst. 15, 32-34 (1962)). Am isoelektrischen Punkt wurden, ebenfalls in Abwesenheit von Zink, kleinere Rhombendodekaeder erhalten, die der kubischen Raumgruppe zuzuordnen sind (Schlichtkrull, Insulin-Kristalle (1961)). Schließlich wurde ebenfalls von Schlichtkrull noch eine monokline Kristallform von Insulin beschrieben, die oberhalb des isoelektrischen Punktes in Gegenwart von Zink und in Gegenwart von Phenol oder Phenolderivaten erhalten wurden. Diese Kristalle wachsen innerhalb einiger Tage zu beachtlicher Größe (bis zu 3 mm) und weisen scharfe Kanten auf. Interessanterweise wurden diese Kristalle nur an Glasflächen, nicht aber an der freien Oberfläche der Lösung gefunden (Schlichtkrull, Insulin-Kristalle, S. 57-60, (1961)).

Die Kristallisation von Insulin aus wäßrigen metallsalzhaltigen Lösungen in Gegenwart von Phenol oder Phenolderivaten ist auch Gegenstand der GB-A 729 670, welche ebenfalls u. a. auf J. Schlichtkrull zurückgeht. Demnach sollen zwar bis dahin noch unbekannte Kristallformen des Insulins (bevorzugt Plättchen, Nadeln und Stäbchen) erhalten werden, für die ein neuer biologischer Effekt geltend gemacht wird ; etwa vom Erhalt monodisperser Suspensionen ist in der GB-A jedoch nirgends die Rede. Solche monodispersen Suspensionen entstehen nach diesem Verfahren auch nicht, wie eigene Versuche gezeigt haben.

Es ist weiterhin u. a. auch bekannt geworden (DE-A 2 018 588), Insulin durch Kristallisation aus einem wäßrigen insulinhaltigen Pankreasextrakt oder aus anderen wäßrigen insulinhaltigen Lösungen zu isolieren und zu reinigen, wobei man die Basizität der insulinhaltigen Lösung auf einen bestimmten pH-Wert (zwischen etwa 7,2 bis etwa 10,0) und eine bestimmte Alkalikationen- oder Ammoniumkationenkonzentration (zwischen etwa 0,2 und etwa 1,0 M) einstellt und dadurch die Kristallisation des Alkali- oder Ammoniuminsulins veranlaßt. Das Salz soll hierbei in einer bestimmten Kristallform (Octadecaeder oder Dodecaeder) anfallen und wasserlöslich sein ; etwa vom Erhalt monodisperser Suspensionen wird auch hier nirgends gesprochen.

Ein verglichen mit Insulin oder Proinsulin unterschiedliches Kristallisationsverhalten findet man bei Insulinderivaten, die in ihrer Nettoladung mindestens eine positive Ladung mehr aufweisen, so daß deren isoelektrischer Punkt zu höheren pH-Werten hin verschoben ist. Dazu zählen insbesondere solche Derivate, die am C-Terminus der B-Kette eine zusätzliche Gruppe basischen Charakters aufweisen, wie etwa die bekanntlich in Pankreasextrakten in kleinen Mengen zu findenden Proinsulin-Abbauprodukte Insulin-Arg$^{B31}$—OH und Insulin-Arg$^{B31}$-Arg$^{32}$—OH. Andere Derivate mit ähnlichen Eigenschaften sind semisynthetisch zugänglich.

Sie weisen beispielsweise andere basische Aminosäuren oder basische Aminosäurederivate (z. B. D-Aminosäuren, Ornithin, Hydroxylysin, Argininol) an Stelle oder zusätzlich zu Arginin oder eine Ester- oder Amidgruppierung mit einfachen Alkoholen oder Aminen am C-Terminus auf. Dabei kann der Alkohol oder das Amid eine zusätzliche positive Ladung mitbringen, wie etwa im Insulin-(B30)-Cholinester.

Es wurde nun überraschenderweise gefunden, daß diese Derivate sich in der Nähe ihres isoelektrischen Punktes aus einem wäßrigen Medium in Gegenwart von Glycerin als Isotoniemittel und einer aromatischen Hydroxyverbindung in Form von sehr gleichmäßig geformten Prismen von etwa 10 µm Größe kristallisieren lassen.

Die Erfindung betrifft daher ein Verfahren zur Herstellung von Suspensionen von gleichmäßig geformten Kristallen mit weitgehend homogener Größenverteilung, insbesondere Prismen von ca. 10 µm Größe, eines oder mehreres Insulin-Derivate der Formel I

(Siehe Formel Seite 3 f.)

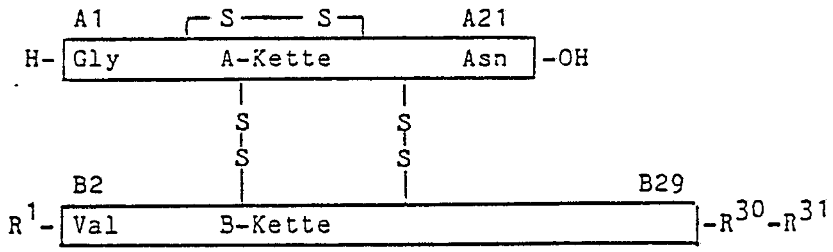


(I)

in welcher

$R^1$ H oder H-Phe bedeutet,

$R^{30}$ für den Rest einer neutralen, genetisch kodierbaren L-Aminosäure steht und

$R^{31}$ für eine physiologisch verträgliche organische Gruppe basischen Charakters mit bis zu 50 C-Atomen steht, an deren Aufbau 0 bis 3 $\alpha$-Aminosäuren beteiligt sind und deren gegebenenfalls vorhandene endständige Carboxyfunktion frei, als Esterfunktion, als Amidfunktion, als Lacton oder zu $CH_2OH$ reduziert vorliegen kann, mit einem isoelektrischen Punkt zwischen 5,8 und 8,5, das dadurch gekennzeichnet ist, daß die Kristallisation in einem wäßrigen Medium in der Nähe des isoelektrischen Punktes des oder der Derivate in Gegenwart von Glycerin als Isotomiemittel und mindestens eines Phenols durchgeführt wird.

Vorzugsweise werden solche Insulin-Derivate der Formel I zur Kristallisation gebracht, in welchen

$R^{31}$ für einen Rest der Formel $—X_nS$ steht, in welchem n = 0, 1, 2 oder 3 ist,

X für gleiche oder verschiedene Reste natürlich vorkommender neutraler oder basischer L-Aminosäuren, vorzugsweise basischer L-Aminosäuren, insbesondere Arg, Lys, His oder Orn und/oder der diesen entsprechenden D-Aminosäuren steht und

S OH oder eine physiologisch verträgliche, die Carboxygruppe blockierende Gruppe bedeutet, die falls n = 0 ist, einen positiv geladenen oder protonierbaren basischen Rest trägt oder, falls n > 0 ist, einen solchen Rest tragen kann und worin der C-Terminus

—X—S auch für den Rest einer zum entsprechenden Alkohol reduzierten Aminosäure oder, im Falle n = 2 oder 3, für den Homoserinlacton-Rest stehen kann.

Besonders bevorzugt sind weiter Insulin-Derivate der Formel I, in welcher $R^1$ für H-Phe und/oder $R^{30}$ für Ala, Thr oder Ser steht und/oder der Rest $R^{31}$ Aminosäurereste bzw. Reste ihrer Derivate (z. B. die entsprechenden Alkohole oder Lactone) ausschließlich in der L-Form enthält.

Genetisch kodierbar sind die folgenden L-Aminosäuren : Gly, Ala, Ser, Thr, Val, Leu, Ile, Asp, Asn, Glu, Gln, Cys, Met, Arg, Lys, His, Tyr, Phe, Trp, Pro (neutrale Aminosäuren unterstrichen).

Unter einer neutralen, natürlich vorkommenden Aminosäure versteht man insbesondere Gly, Ala, Ser, Thr, Val, Leu, Ile, Asn, Gln, Cys, Met, Tyr, Phe, Pro oder Hyp. Unter einer basischen, natürlich vorkommenden Aminosäure versteht man insbesondere Arg, Lys, Hyl, Orn, Cit oder His.

Insbesondere kommen auch solche Peptide der Formel I in Betracht, deren C-Terminus eine die Carboxygruppe blockierende Gruppe aus der Reihe ($C_1$ bis $C_6$)-Alkoxy, ($C_3$ bis $C_6$)-Cycloalkyloxy, $NH_2$, ($C_1$ bis $C_6$)-Alkylamino, Di-($C_1$ bis $C_6$)-alkylamino, Amino-($C_1$ bis $C_6$)-alkoxy, ($C_1$ bis $C_4$)-Alkylamino-($C_2$ bis $C_6$)-alkoxy, Di($C_1$ bis $C_4$)-ammonio-($C_2$ bis $C_6$)-alkoxy, Tri($C_1$ bis $C_4$)-ammonio-($C_2$ bis $C_6$)-alkoxy, Amino-($C_2$ bis $C_6$)-alkylamino, [($C_1$ bis $C_4$)-Alkylamino]-($C_2$ bis $C_6$)-alkylamino, [Di($C_1$ bis $C_4$)-alkylamino]-($C_2$ bis $C_6$)-alkylamino oder [Tri($C_1$ bis $C_4$) alkylammonio]-($C_2$ bis $C_6$)-alkylamino trägt.

Basische, die Carboxygruppe blockierende Gruppen sind insbesondere $—O—[CH_2]_p—NR_2$, $—O—[CH_2]_p—\overset{\oplus}{N}R_3$, $—NH—[CH_2]_p—NR_2$ oder $—NH—[CH_2]_p—\overset{\oplus}{N}R_3$, worin p = 2 bis 6 ist und R gleich oder verschieden ist und für Wasserstoff oder ($C_1$ bis $C_4$)-Alkyl steht.

Die Insulin-Derivate der Formel I weisen bevorzugt die Sequenz von Human-, Schweine- oder Rinderinsulin auf.

Beispielsweise lassen sich mit Hilfe des erfindungsgemäßen Verfahrens Kristallsuspensionen nachstehender Insulin-Derivate erhalten (ohne die Erfindung auf diese zu beschränken) :

Humaninsulin-$Arg^{31}$—OH
Humaninsulin-$Arg^{31}$-$Ala^{B32}$—OH
Schweineinsulin-$Arg^{B31}$—OH
Schweineinsulin-$Arg^{B31}$-$Ala^{B32}$—OH
Rinderinsulin-$Arg^{B31}$—OH
Rinderinsulin-$Arg^{B31}$-$Arg^{B32}$—OH
Des-$Phe^{B1}$-Schweineinsulin-$Arg^{B21}$—OH
Des-$Phe^{B1}$-Humaninsulin-$Arg^{B31}$—OH
Des-$Phe^{B1}$-Schweineinsulin-$Arg^{B31}$-$Arg^{B32}$—OH
Des-$Phe^{B1}$-Humaninsulin-$Arg^{B31}$-$Arg^{B32}$—OH
Schweineinsulin-$Arg^{B31}$—$OCH_3$
Humaninsulin-$Arg^{B31}$—$OCH_3$
Rinderinsulin-$Arg^{B31}$—$OCH_3$

Schweineinsulin-Arg$^{B31}$-Arg$^{B32}$—$OCH_3$
Humaninsulin-Arg$^{B31}$-Arg$^{B32}$—$OCH_3$
Des-Thr$^{B30}$-Humaninsulin-Val$^{B30}$-Arg$^{B31}$—OH
Des-Thr$^{B30}$-Humaninsulin-Val$^{B30}$-Ala$^{B31}$-Arg$^{B32}$—OH
Humaninsulin-Lys$^{B31}$—OH
Humaninsulin-D-Arg$^{B31}$—OH
Humaninsulin-D-Arg$^{B31}$-Arg$^{B32}$—OH
Humaninsulin-Arg$^{B31}$-D-Arg$^{B32}$—OH
Humaninsulin-Lys$^{B31}$-Arg$^{B32}$—OH
Humaninsulin-Arg$^{B31}$-Lys$^{B32}$—OH
Humaninsulin-Argininol$^{B31}$
Humaninsulin-Val$^{B31}$-Arg$^{B32}$—OH
Humaninsulin-Val$^{B31}$-Arg$^{B32}$-Arg$^{B33}$—OH
Humaninsulin-Arg$^{B31}$-Argininol$^{B32}$
Humaninsulin-Lys$^{B31}$-Arg$^{B32}$-Arg$^{B33}$—OH

Humaninsulin-Arg$^{B31}$ 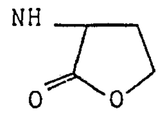

Humaninsulin-Arg$^{B31}$-Arg$^{B32}$ - 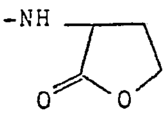

Humaninsulin-Arg$^{B31}$—$NH_2$
Humaninsulin-Arg$^{B31}$-Arg$^{B32}$—$NH_2$
Humaninsulin-Orn$^{B31}$—OH
Humaninsulin-Leu$^{B31}$-Cit$^{B32}$—OH
Humaninsulin-(B30)—$OCH_2CH_2$—$NH_2$
Humaninsulin-(B30)—NH—$CH_2CH_2$—$NH_2$
Humaninsulin-Arg$^{B31}$—O—$CH_2$—$CH_2$—$NH_2$
Humaninsulin-Arg$^{B31}$—$CH_2$—$CH_2$—$N(CH_3)_2$
Humaninsulin-(B30)—O—$CH_2$—$CH_2$—$\overset{\oplus}{N}(CH_3)_3$
Humaninsulin-(B30)—NH—$CH_2$—$CH_2$—$\overset{\oplus}{N}(CH_3)_3$
Humaninsulin-Leu$^{B31}$—O—$CH_2$—$CH_2$—$CH_2$—$\overset{\oplus}{N}(C_2H_5)_3$
Humaninsulin-Trp$^{B31}$-Trp$^{B32}$-Trp$^{B33}$—$NH(CH_2)_6$—$\overset{\oplus}{N}(n\text{-}Bu)_3$

Die Insulin-Derivate der Formel I werden hergestellt, indem man

a) ein Des-Octapeptid (B23-30)-Insulin der Formel II,

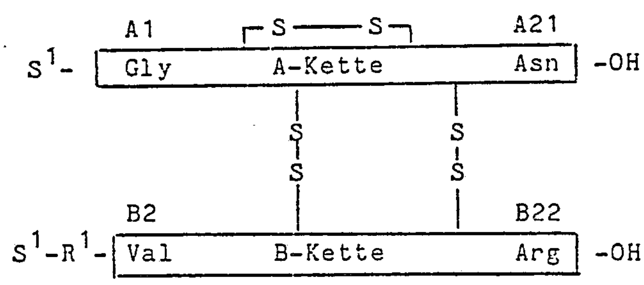

(II)

in der $R^1$ Phe oder eine Bindung und $S^1$ eine protonensolvolytisch oder durch β-Eliminierung abspaltbare Aminoschutzgruppe wie den tert-Butyloxycarbonyl-(Boc), den tert-Amyloxycarbonyl-(Aoc) oder den Methylsulfonylethyloxycarbonyl-(Msc)-rest bedeuten, kondensiert mit einem Peptid der Formel III

$$H—Gly—Phe—Phe—Tyr(S^2)—Thr(S^2)—Pro—Lys(S^3)—R^{30}—R^{31} \quad (III)$$

in welcher $R^{30}$ und $R^{31}$ die oben definierten Bedeutungen haben, $S^2$ für Wasserstoff, Bzl oder $Bu^t$ und $S^3$ für eine Urethanschutzgruppe, wie Boc, Moc, Fmoc oder Z stehen, wobei in den Resten $R^{30}$ und $R^{31}$ gegebenenfalls vorhandene freie COOH—, OH—, SH—, ω—$NH_2$—, Guanidino- und/oder Imidazol-Gruppen, falls erforderlich, in an sich bekannter Weise geschützt vorliegen und anschließend gegebenenfalls vorhandene Schutzgruppen in an sich bekannter Weise abspaltet,

b) ein Des-B30-Insulin der Formel I, in welcher $R^1$ für H oder H-Phe und der C-Terminus $R^{30}$-$R^{31}$ zusammen für OH stehen, in Gegenwart von Trypsin oder einer trypsinähnlichen Endopeptidase umsetzt mit einer Verbindung der Formel IV

$$H—R^{30}—R^{31} \qquad (IV)$$

in der $R^{30}$ und $R^{31}$ die oben definierten Bedeutungen haben und worin vorhandene freie COOH—, OH—, SH—, $\omega$—$NH_2$—, Guanidino- und/oder Imidazol-Funktionen, falls erforderlich, in an sich bekannter Weise geschützt vorliegen und anschließend gegebenenfalls vorhandene Schutzgruppen in an sich bekannter Weise abspaltet, oder

c) zur Herstellung eines Insulin-Derivates mit L-konfigurierten Aminosäureresten in $R^{31}$ ein Proinsulin, Proinsulinderivat Präproinsulin oder Präproinsulinderivat oder ein Intermediat dieser Verbindungen chemisch und/oder enzymatisch spaltet.

Des-$Phe^{B1}$-Insuline als Ausgangsverbindungen sind beispielsweise aus der DE-PS 20 05 658 oder aus der EP-A-46 979 bekannt.

Die bei der Verfahrensvariante b) als Ausgangsverbindungen verwendeten Des-B30-Insuline sind beispielsweise aus der EP-A-46 979 oder Hoppe-Seyler's Z. Physiol. Chem. 359/1978/799 bekannt. Das bei Variante b) verwendete Ausgangsmaterial der Formel IV wird in an sich bekannter Weise nach den Methoden der Peptidchemie hergestellt. Brauchbare Schutzgruppen für IV sind detailiert beschrieben bei M. Bodanzky et al., Peptide Synthesis, Ind. Ed. 1976, Wiley & Sons.

Durch gentechnologische Methoden sind inzwischen humanes oder Primaten-Proinsulin als Ausgangsmaterial für die Verfahrensvariante c) zugänglich. Die Derivate Arg(B31) und Di-Arg(B31-32) sind daraus durch einfache Verdauung mit Trypsin oder trypsin-ähnlichen Enzymen zugänglich. Daneben lassen sich aber auch relativ einfach Plasmide konstruieren, die durch Spaltung entsprechender Präproinsulinderivate zu neuen Insulinderivaten führen, weil sie an Stelle der natürlich an B31 oder B32 befindlichen Arginine für andere neutrale oder basische Aminosäuren codieren.

Die Herstellung von Proinsulin unter Anwendung der Rekombinations-DNA-Methodologie erfordert die Bildung einer DNA-Sequenz für die Aminosäuresequenz eines Proinsulins, was sich entweder durch Isolierung, Konstruktion oder eine Kombination aus beidem erreichen läßt. Die Proinsulin-DNA wird dann in Lesephase in einen geeigneten Clonierungs- und Expressionsträger eingesetzt. Der Träger dient zur Transformierung eines geeigneten Mikroorganismus, und der hierbei erhaltene transformierte Mikroorganismus wird dann Fermentationsbedingungen unterzogen, die zur Bildung weiterer Kopien des proinsulingenhaltigen Vektors und zur Expression von Proinsulin einem, Reininsulinderivat oder einem Proinsulinvorläufer bzw. einem Präproinsulinderivat führen.

Handelt es sich beim Expressionsprodukt um einen Proinsulinvorläufer, dann enthält ein solches Produkt im allgemeinen die Proinsulinaminosäuresequenz, die an ihrer endständigen Aminogruppe an ein Bruchstück eines Proteins gebunden ist, das normalerweise durch die der Gensequenz ausgedrückt wird, bei welcher das Proinsulin oder Proinsulinderivat eingesetzt worden ist. Die Proinsulinaminosäuresequenz ist an das Proteinbruchstück über eine spezifisch spaltbare Stelle gebunden, bei der es sich beispielsweise um Methionin handelt. Die erhaltene Proinsulinaminosäuresequenz wird vom verschmolzenen Genprodukt, beispielsweise wie in der DE-A-32 32 036 beschrieben, abgespalten und das Proinsulin nach Reinigung isoliert.

Hinsichtlich der einzelnen Bedingungen des erfindungsgemäßen Verfahrens ist es vorteilhaft, wenn wenigstens eines der folgenden Merkmale erfüllt ist:

Vorhandensein einer Zinkmenge von bis zu 1 %, bezogen auf die Insulinmasse, im Kristallisationsmedium,

Kristallisation bei Temperaturen von 3 bis 27 °C,

Konzentration des Insulin-Derivates (I) im Kristallisationsmedium 0,2 bis 40 mg/ml,

Durchführung der Kristallisation innerhalb eines pH-Bereichs von 1 pH-Einheit unterhalb des isoelektrischen Punktes bis 1 pH-Einheit oberhalb dieses Punktes und

Verwendung von Monohydroxybenzol und/oder Kresol als Phenol.

Insbesondere soll die Zinkmenge im Kristallisationsmedium nicht über 0,8 %, bezogen auf die Insulin(-Derivate)-Masse, betragen. Gewöhnlich genügen zur Kristallisation nur relativ geringe Zinkmengen von maximal 40 µg/100 Einheiten, bevorzugt aber nicht über 30 µg/100 Einheiten, die u. U. bereits in der Trockensubstanz enthalten sein können.

Als Temperaturbereich für die Kristallisation ist der Bereich zwischen 10 und 20 °C besonders günstig.

Ein besonders bevorzugter Konzentrationsbereich für das Insulin-Derivat (I) im Kristallisationsmedium liegt bei 1 bis 7,5 mg/ml.

Innerhalb des pH-Bereichs von 1 pH-Einheit unterhalb des isoelektrischen Punktes bis 1 pH-Einheit

oberhalb dieses Punktes ($P_I \pm 1$) ist im Prinzip kein engerer Bereich besonders bevorzugt.

Ebenso ist auch unter den beiden Phenolverbindungen Monohydroxybenzol und Kresol keine besonders bevorzugt.

Die Suspension kann während des Verfahrens schwach bewegt (z. B. in einem Rührgefäß gerührt) werden.

Die Kristalle können beispielsweise auch in einem Medium erhalten werden, welches bereits der fertigen Injektionssuspension (bei reinen Verzögerungspräparaten) entspricht. Das (sterile) Medium kann demgemäß bereits neben Phenol oder ähnlichen Aromaten und eventuell Zink, sowie Glycerin als Isotoniemittel noch eine Puffersubstanz enthalten, die auch zur pH-Einstellung verwendet werden kann. Bevorzugte Puffersubstanzen sind Phosphat (z. B. Natriumphosphat-) oder Citrat-Puffer.

Die Erfindung betrifft weiterhin eine Kristallsuspension eines oder mehrerer Insulin-Derivate der Formel I, erhältlich nach dem vorher erwähnten Kristallisationsverfahren.

Die erfindungsgemäßen Kristallsuspensionen können zusätzlich mindestens eine Verbindung der Gruppe a) Insulin-Derivate der Formel I in gelöster und/oder amorpher Form, b) Insulin, c) Proinsulin und d) C-Peptid, jeweils in gelöster, amorpher und/oder kristalliner Form und ggf. weiterhin zusätzlich ein Hilfsmittel mit verzögernder Wirkung enthalten. Als Hilfsmittel mit verzögernder Wirkung (auf die Insulinfreisetzung) kommen z. B. Globin oder Protaminsulfat in Frage.

Neben den Vorteilen hinsichtlich Kristallgröße und deren Homogenität bedingt der relativ geringe Zinkgehalt, der unterhalb der Konzentration liegt, wo Zink als Depotträger zu betrachten ist, daß die erfindungsgemäßen Kristallsuspensionen frei mit gelöstem Insulin mischbar sind. Es ist somit beispielsweise möglich, Insulinlösungen mit Suspensionen der Insulin-Derivat-Kristalle vor Applikation zu vereinigen. Durch Variation der Anteile der einzelnen Komponenten läßt sich das Wirkprofil des so erhaltenen Arzneimittels optimal steuern.

Wäre eine höhere Zinkkonzentration zur Stabilität der Kristalle nötig (wie dies etwa bei den Zink-Insulin-Rhomben der Fall ist), so würde zugegebenes, gelöstes Insulin ausfallen und nicht mehr als schnell wirkende Komponente wirksam werden.

Somit weisen Kristallsuspensionen aus den beschriebenen Derivaten in idealer Weise jene Eigenschaften auf, die zur Behandlung des Diabetes mellitus wünschenswert sind. Die Erfindung bezieht sich daher auch auf eine solche Kristallsuspension bzw. solche Kristallsuspensionen zur Verwendung als Heilmittel, insbesondere bei der Behandlung von Diabetes mellitus, sowie weiterhin auf eine Verbindung der Formel I in Form gleichmäßig geformter Kristalle mit weitgehend homogener Größenverteilung, insbesondere Prismen von etwa 10 µm Größe, erhältlich nach einem Verfahren der vorstehend beschriebenen Art.

Das Verzögerungsprinzip ist den Insulin-Derivaten inhärent und geht auf ein proteinchemisches Phänomen, die Schwerlöslichkeit am isoelektrischen Punkt, zurück.

Zur weiteren Erläuterung der Erfindung sollen die folgenden Beispiele dienen :

## Beispiel 1

Kristallisation einer Mischung aus 50 % Humaninsulin-Arg$^{B31}$—OH, hergestellt durch tryptische Spaltung aus gentechnologisch gewonnenem Präproinsulin und 50 % Humaninsulin-B30-cholinester ; hergestellt durch Semisynthese aus Schweineinsulin in einer Zubereitung mit 40 I.E./ml und deren verzögerte Wirkung.

In Wasser werden vermischt :

| | |
|---|---|
| Humaninsulin-Arg$^{B31}$—OH (27,5 I.E./mg) | 7,3 mg |
| Humaninsulin-(B30)-cholinester (28,0 I.E./mg) | 7,1 mg |
| Natriumdihydrogenphosphat-di-hydrat | 21,0 mg |
| m-Kresol | 15,0 mg |
| Phenol | 0,6 mg |
| Glycerin | 16,0 mg |

Mit verdünnter NaOH wird pH = 7,0 eingestellt und mit Wasser auf ein Gesamt-Volumen von 10 ml aufgefüllt. Der Ansatz wird bei Raumtemperatur leicht bewegt, wobei prismenförmige Kristalle von ca. 10 µm Größe entstehen. Eine solche Suspension zeigt am Kaninchen bei einer Dosierung von 4,0 I.E./kg eine stark verzögerte Wirkung. Die Blutglucosewerte (in % des Ausgangswertes) sind (n = 12 Tiere) :

| t(h) | 0 | 1 | 2 | 4 | 6 |
|---|---|---|---|---|---|
| Blutglucose | 100 % | 51 % | 44 % | 69 % | 83 % |

## Beispiel 2

Suspension aus kristallinem Humaninsulin-$Arg^{B31}$-Lys-$^{B32}$—$OCH_3$ hergestellt durch Semisynthese aus Schweineinsulin, in Mischung mit 50 % Isophan-Protamin-Humaninsulinkristallen mit 100 I.E./ml.

Man löst in einem Gesamt-Volumen von 10 ml in Wasser :

| | |
|---|---|
| Humaninsulin-$Arg^{B31}$-$Lys^{B31}$—$OCH_3$ (27,0 I.E./mg), zinkfrei | 37,0 mg |
| Zinkchlorid | 0,6 mg |
| Natriumdihydrogenphosphat-dihydrat | 21,0 mg |
| m-Kresol | 15,0 mg |
| Phenol | 6,0 mg |
| Glycerin | 160,0 mg |

Mit 1 N HCl bzw. 1N NaOH wird pH = 7,4 eingestellt. Der Ansatz wird bei Raumtemperatur schwach gerührt, wobei innerhalb eines Tages prismenförmige Kristalle entstehen. Getrennt werden Isophan-Humaninsulinkristalle (NPH) hergestellt, indem man in Wasser mischt :

| | |
|---|---|
| Humaninsulin (28,0 I.E./mg) | 35,7 mg |
| Protaminsulfat | 3,2 mg |
| Natriumdihydrogenphosphat-dihydrat | 21,0 mg |
| m-Kresol | 15,0 mg |
| Phenol | 6,0 mg |
| Glycerin | 160,0 mg |

Mit 1 N HCl bzw. 1N NaOH wird pH = 7,4 eingestellt und mit Wasser auf 10 ml ergänzt. Der Ansatz wird schwach gerührt, wobei die typischen stäbchenförmigen NPH-Kristalle entstehen.

Die beiden Suspensionen werden zusammengegeben, wobei das gewünschte Mischpräparat mit 100 I.E./ml entsteht, das eine stark verzögerte Wirkung im Tierversuch zeigt.

## Beispiel 3

Depot-Präparat mit 40 I.E./ml aus 75 % kristallinem Humaninsulin-$Arg^{B31}$-$Arg^{B32}$—OH, gewonnen durch tryptische Spaltung aus gentechnologisch gewonnenem Präproinsulin, und 25 % Humaninsulin in gelöster Form.

Man löst in 250 ml verdünnter HCl :

1,111 g Humaninsulin-$Arg^{B31}$-$Arg^{B32}$—OH (27,0 I.E./mg) so, daß der pH = 4,0 beträgt. Diese Lösung wird in geeigneter Weise sterilfiltriert.

Getrennt löst man in Wasser :

| | |
|---|---|
| Natriumdihydrogenphosphat-dihydrat | 1,575 g |
| m-Kresol | 1,125 g |
| Phenol | 0,450 g |
| Glycerin | 14,118 g |

Mit 1 N NaOH wird pH = 7,5 eingestellt und mit Wasser auf 0,5 l ergänzt. Diese Pufferlösung wird ebenfalls in geeigneter Weise sterilfiltriert.

Unter aseptischen Bedingungen werden unter Rühren die beiden sterilen Lösungen zusammengegeben und so lange gerührt, bis homogene Kristalle von ca. 10 µm Größe entstanden sind (A). Eine Insulinlösung (B) wird hergestellt, indem man vermischt :

| | |
|---|---|
| Humaninsulin (28,0 I.E./mg) | 0,357 g |
| Natriumdihydrogenphosphat-dihydrat | 0,525 g |
| m-Kresol | 0,375 g |
| Phenol | 0,150 g |
| Glycerin | 4,706 g |

Die Lösung wird mit verdünnter NaOH auf pH = 7,3 gebracht und mit Wasser auf 0,25 l ergänzt. Diese Lösung wird sterilfiltriert. Unter aseptischen Bedingungen wird die Insulinlösung (B) zu der Kristallsuspension (A) gegeben und das Präparat in geeignete Mehrfachentnahmeflaschen abgefüllt.

Ein solches Depotpräparat kann für die Behandlung des Diabetes mellitus verwendet werden.

**Patentansprüche** (für die Vertragsstaaten : BE, CH, DE, FR, GB, IT, LI, LU, NL, SE)

1. Verfahren zur Herstellung von Suspensionen von gleichmäßig geformten Kristallen mit weitgehend homogener Größenverteilung, insbesondere Prismen von ca. 10 µm Größe, eines oder mehrerer Insulin-Derivate der Formel I,

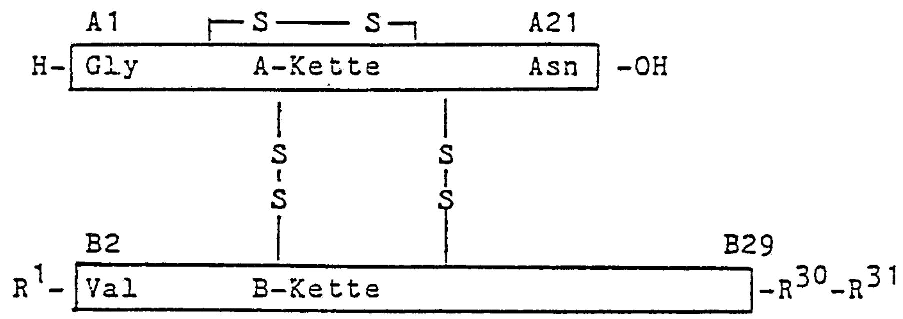

(I)

in welcher

$R^1$ H oder H-Phe bedeutet,

$R^{30}$ für den Rest einer neutralen, genetisch kodierbaren L-Aminosäure steht und

$R^{31}$ für eine physiologisch verträgliche organische Gruppe basischen Charakters mit bis zu 50 C-Atomen steht, an deren Aufbau 0 bis 3 α-Aminosäuren beteiligt sind und deren gegebenenfalls vorhandene endständige Carboxyfunktion frei, als Esterfunktion, als Amidfunktion, als Lacton oder zu $CH_2OH$ reduziert vorliegen kann, mit einem isoelektrischen Punkt zwischen 5,8 und 8,5,
dadurch gekennzeichnet, daß die Kristallisation in einem wäßrigen Medium in der Nähe des isoelektrischen Punktes des oder der Derivate in Gegenwart von Glycerin als Isotoniemittel und mindestens eines Phenols durchgeführt wird.

2. Verfahren gemäß Anspruch 1, dadurch gekennzeichnet, daß in Formel I

$R^{31}$ für einen Rest der Formel —$X_n$S steht, in welchem n = 0, 1, 2 oder 3 ist,

X für gleiche oder verschiedene Reste natürlich vorkommender neutraler oder basischer L-Aminosäuren, vorzugsweise basischer L-Aminosäuren, insbesondere Arg, Lys, His, Hyl, Cit oder Orn und/oder der diesen entsprechenden D-Aminosäuren steht und

S OH oder eine physiologisch verträgliche, die Carboxygruppe blockierende Gruppe bedeutet, die falls n = 0 ist, einen positiv geladenen oder protonierbaren basischen Rest trägt oder, falls n > 0 ist, einen solchen Rest tragen kann und worin der C-Terminus

—X—S auch für den Rest einer zum entsprechenden Alkohol reduzierten Aminosäure oder, im Falle n = 2 oder 3, für den Homoserinlacton-Rest stehen kann.

3. Verfahren gemäß Anspruch 1 oder 2, gekennzeichnet durch wenigstens eines der Merkmale, daß in Formel I $R^1$ für H-Phe steht, daß $R^{30}$ für Ala, Thr oder Ser steht und daß der Rest $R^{31}$ Aminosäurereste bzw. Reste ihrer Derivate ausschließlich in der L-Form enthält.

4. Verfahren nach einem oder mehreren der Ansprüche 1 bis 3, gekennzeichnet durch wenigstens eines der Merkmale, daß im Kristallisationsmedium eine Zinkmenge von bis zu 1 %, bezogen auf die Insulinmasse, vorhanden ist, daß die Kristallisation bei Temperaturen von 3-27 °C erfolgt, daß die Konzentration des Insulinderivats (I) im Kristallisationsmedium 0,2 bis 40 mg/ml beträgt, daß die Kristallisation innerhalb eines pH-Bereich von 1 pH-Einheit unterhalb des isoelektrischen Punktes bis 1 pH-Einheit oberhalb dieses Punktes durchgeführt wird und daß das Phenol das Monohydroxybenzol und/oder Kresol ist.

5. Verfahren nach einem oder mehreren der Ansprüche 1 bis 4, dadurch gekennzeichnet, daß in Gegenwart eines Phosphat- oder Citratpuffers gearbeitet wird.

6. Kristallsuspension eines oder mehrerer Insulin-Derivate der Formel I, erhältlich durch ein Verfahren nach einem oder mehreren der Ansprüche 1 bis 5.

7. Kristallsuspension gemäß Anspruch 6, dadurch gekennzeichnet, daß diese zusätzlich mindestens eine Verbindung der Gruppe a) Insulin-Derivate der Formel I in gelöster und/oder amorpher Form, b) Insulin, c) Proinsulin und d) C-Peptid jeweils in gelöster, amorpher und/oder kristalliner Form und gegebenenfalls zusätzlich ein Hilfsmittel mit verzögernder Wirkung enthält.

8. Kristallsuspension gemäß Anspruch 6 oder 7 zur Verwendung als Heilmittel.

9. Kristallsuspensionen gemäß einem der Ansprüche 6 oder 7 zur Verwendung als Heilmittel bei der Behandlung des Diabetes mellitus.

10. Verbindung der Formel I in Form gleichmäßig geformter Kristalle mit weitgehend homogener Größenverteilung, insbesondere Prismen von etwa 10 µm Größe, erhältlich durch ein Verfahren nach einem oder mehreren der Ansprüche 1 bis 5.

**Patentansprüche** (für den Vertragsstaat AT)

1. Verfahren zur Herstellung von Suspensionen von gleichmäßig geformten Kristallen mit weitgehend

homogener Größenverteilung, insbesondere Prismen von ca. 10 µm Größe, eines oder mehrerer Insulin-Derivate der Formel I,

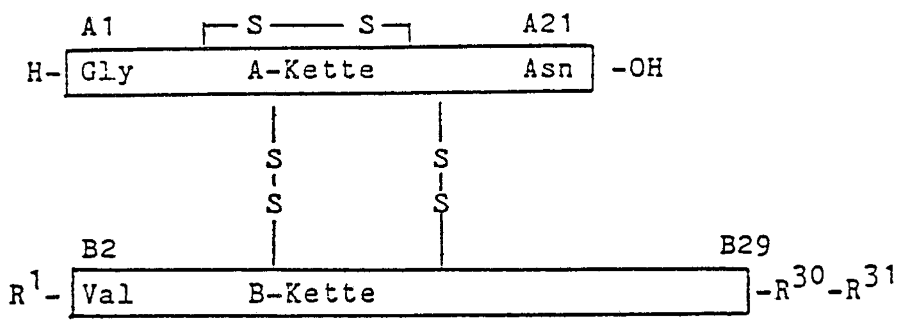

(I)

in welcher

$R^1$ H oder H-Phe bedeutet,

$R^{30}$ für den Rest einer neutralen, genetisch kodierbaren L-Aminosäure steht und

$R^{31}$ für eine physiologisch verträgliche organische Gruppe basischen Charakters mit bis zu 50 C-Atomen steht, an deren Aufbau 0 bis 3 α-Aminosäuren beteiligt sind und deren gegebenenfalls vorhandene endständige Carboxyfunktion frei, als Esterfunktion, als Amidfunktion, als Lacton oder zu $CH_2OH$ reduziert vorliegen kann, mit einem isoelektrischen Punkt zwischen 5,8 und 8,5, dadurch gekennzeichnet, daß die Kristallisation in einem wäßrigen Medium in der Nähe des isoelektrischen Punktes des oder der Derivate in Gegenwart von Glycerin als Isotoniemittel und mindestens eines Phenols durchgeführt wird.

2. Verfahren gemäß Anspruch 1, dadurch gekennzeichnet, daß in Formel I

$R^{31}$ für einen Rest der Formel $—X_nS$ steht, in welchem n = 0, 1, 2 oder 3 ist,

X für gleiche oder verschiedene Reste natürlich vorkommender neutraler oder basischer L-Aminosäuren, vorzugsweise basischer L-Aminosäuren, insbesondere Arg, Lys, His, Hyl, Cit oder Orn und/oder der diesen entsprechenden D-Aminosäuren steht und

S OH oder eine physiologisch verträgliche, die Carboxygruppe blockierende Gruppe bedeutet, die falls n = 0 ist, einen positiv geladenen oder protonierbaren basischen Rest trägt oder, falls n > 0 ist, einen solchen Rest tragen kann und worin der C-Terminus

—X—S auch für den Rest einer zum entsprechenden Alkohol reduzierten Aminosäure oder, im Falle n = 2 oder 3, für den Homoserinlacton-Rest stehen kann.

3. Verfahren gemäß Anspruch 1 oder 2, gekennzeichnet durch wenigstens eines der Merkmale, daß in Formel I $R^1$ für H-Phe steht, daß $R^{30}$ für Ala, Thr oder Ser steht und daß der Rest $R^{31}$ Aminosäurereste bzw. Reste ihrer Derivate ausschließlich in der L-Form enthält.

4. Verfahren nach einem oder mehreren der Ansprüche 1 bis 3, gekennzeichnet durch wenigstens eines der Merkmale, daß im Kristallisationsmedium eine Zinkmenge von bis zu 1 %, bezogen auf die Insulinmasse, vorhanden ist, daß die Kristallisation bei Temperaturen von 3-27 °C erfolgt, daß die Konzentration des Insulinderivats (I) im Kristallisationsmedium 0,2 bis 40 mg/ml beträgt, daß die Kristallisation innerhalb eines pH-Bereich von 1 pH-Einheit unterhalb des isoelektrischen Punktes bis 1 pH-Einheit oberhalb dieses Punktes durchgeführt wird und daß das Phenol das Monohydroxybenzol und/oder Kresol ist.

5. Verfahren nach einem oder mehreren der Ansprüche 1 bis 4, dadurch gekennzeichnet, daß in Gegenwart eines Phosphat- oder Citratpuffers gearbeitet wird.

6. Verfahren gemäß einem oder mehreren der Ansprüche 1 bis 5, dadurch gekennzeichnet, daß die hergestellten Kristallsuspensionen zusätzlich mindestens eine Verbindung der Gruppe a) Insulin-Derivate der Formel I in gelöster und/oder amorpher Form, b) Insulin, c) Proinsulin und d) C-Peptid jeweils in gelöster, amorpher und/oder kristalliner Form und gegebenenfalls zusätzlich ein Hilfsmittel mit verzögernder Wirkung enthält.

7. Verfahren zur Herstellung von Kristallsuspensionen gemäß einem oder mehreren der Ansprüche 1 bis 6 zur Verwendung als Heilmittel.

8. Verfahren zur Herstellung von Kristallsuspensionen gemäß einem oder mehreren der Ansprüche 1 bis 6 zur Verwendung als Heilmittel bei der Behandlung von Diabetes mellitus.

**Claims** (for the Contracting States : BE, CH, DE, FR, GB, IT, LI, LU, NL, SE)

1. Process for the preparation of suspensions of uniformly shaped crystals having a substantially homogeneous size distribution, in particular prisms about 10 µm in size, of one or more insulin derivatives of the formula I

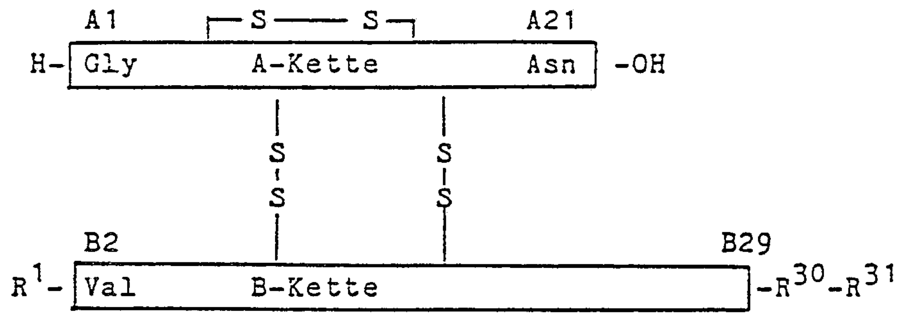

(I)

in which

$R^1$ denotes H or H-Phe,

$R^{30}$ represents the residue of a neutral, genetically encodable L-amino acid and

$R^{31}$ represents a physiologically tolerated organic group which is basic in character and has up to 50 C atoms, and in whose structure 0 to 3 α-amino acids are involved, and whose terminal carboxyl group, which is present where appropriate, can be in the free form, or in the form of an ester group, an amide group, a lactone or reduced to $CH_2OH$, having an isoelectric point between 5.8 and 8.5,

characterized in that the crystallization is carried out in an aqueous medium in the neighbourhood of the isoelectric point of the derivative(s) in the presence of glycerol as tonicizing agent and at least one phenol.

2. Process according to claim 1, characterized in that in formula I

$R^{31}$ represents a radical of the formula $-X_nS$ in which n is 0, 1, 2 or 3,

X represents identical or different residues of naturally occurring neutral or basic L-amino acids, preferably basic L-amino acids, in particular Arg, Lys, His, Hyl, Cit or Orn and/or of the D-amino acids corresponding to them, and

S denotes OH or a physiologically tolerated group which blocks the carboxyl group and, if n is 0, carries a basic radical which is positively charged or can be protonated or, if $n > 0$, can carry a radical of this type, and in which the C terminal

—X—S can also represent the residue of an amino acid which has been reduced to the corresponding alcohol or, in the case $n = 2$ or 3, can represent the homoserine lactone residue.

3. Process according to claim 1 or 2, characterized by at least one of the features that in formula I $R^1$ represents H-Phe, that $R^{30}$ represents Ala, Thr or Ser, and that the radical $R^{31}$ contains amino acid residues or residues of their derivatives exclusively in the L form.

4. Process according to one or more of claims 1 to 3, characterized by at least one of the features that an amount of zinc of up to 1 % relative to the mass of insulin is present in the crystallization medium, that the crystallization is carried out at temperatures of 3-27 °C, that the concentration of the insulin derivative (I) in the crystallization medium is 0.2 to 40 mg/ml, that the crystallization is carried out within a pH range from 1 pH unit below the isoelectric point to 1 pH unit above this point, and that the phenol is the monohydroxybenzene and/or cresol.

5. Process according to one or more of claims 1 to 4, characterized in that it is carried out in the presence of a phosphate or citrate buffer.

6. Crystal suspension of one or more insulin derivatives of formula I, obtainable by a process according to one or more of claims 1 to 5.

7. Crystal suspension according to claim 6, characterized in that the latter additionally contains at least one compound of the group comprising a) insulin derivatives of the formula I in dissolved and/or amorphous form, b) insulin, c) proinsulin and d) C-peptide, in each case in dissolved, amorphous and/or crystalline form and, where appropriate, additionally an auxiliary having a delaying action.

8. Crystal suspension according to claim 6 or 7 for use as medicine.

9. Crystal suspensions according to one of claims 6 or 7 for use as medicines in the treatment of diabetes mellitus.

10. Compound of the formula I in the form of uniformly shaped crystals having a substantially homogeneous size distribution, in particular prisms about 10 µm in size, obtainable by a process according to one or more of claims 1 to 5.

**Claims** (for the Contracting State AT)

1. Process for the preparation of suspensions of uniformly shaped crystals having a substantially homogeneous size distribution, in particular prisms about 10 µm in size, of one or more insulin derivatives of the formula I

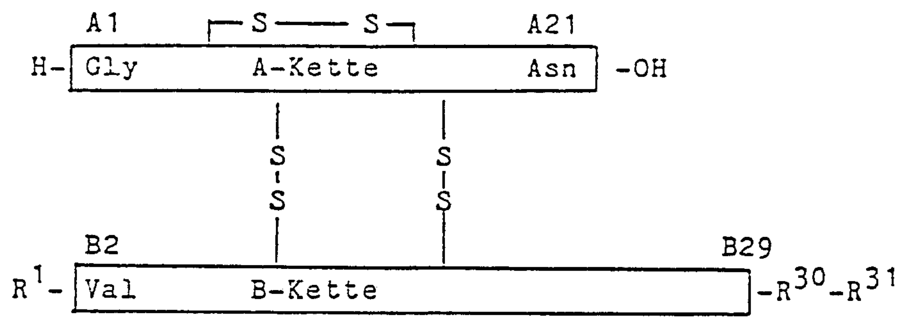

(I)

in which

$R^1$ denotes H or H-Phe,

$R^{30}$ represents the residue of a neutral, genetically endocable L-amino acid and

$R^{31}$ represents a physiologically tolerated organic group which is basic in character and has up to 50 C atoms, and in whose structure 0 to 3 α-amino acids are involved, and whose terminal carboxyl group, which is present where appropriate, can be in the free form, or in the form of an ester group, an amide group, a lactone or reduced to $CH_2OH$, having an isoelectric point between 5.8 and 8.5, characterized in that the crystallization is carried out in an aqueous medium in the neighbourhood of the isoelectric point of the derivative(s) in the presence of glycerol as tonicizing agent and at least one phenol.

2. Process according to claim 1, characterized in that in formula I

$R^{31}$ represents a radical of the formula $—X_nS$ in which n is 0, 1, 2 or 3,

X represents identical or different residues of naturally occurring neutral or basic L-amino acids, preferably basic L-amino acids, in particular Arg, Lys, His, Hyl, Cit or Orn and/or of the D-amino acids corresponding to them, and

S denotes OH or a physiologically tolerated group which blocks the carboxyl group and, if n is 0, carries a basic radical which is positively charged or can be protonated or, if $n > 0$, can carry a radical of this type, and in which the C terminal

—X—S can also represent the residue of an amino acid which has been reduced to the corresponding alcohol or, in the case $n = 2$ or 3, can represent the homoserine lactone residue.

3. Process according to claim 1 or 2, characterized by at least one of the features that in formula I $R^1$ represents H-Phe, that $R^{30}$ represents Ala, Thr or Ser, and that the radical $R^{31}$ contains amino acid residues or residues of their derivatives exclusively in the L form.

4. Process according to one or more of claims 1 to 3, characterized by at least one of the features that an amount of zinc of up to 1 % relative to the mass of insulin is present in the crystallization medium, that the crystallization is carried out at temperatures of 3-27 °C, that the concentration of the insulin derivative (I) in the crystallization medium is 0.2 to 40 mg/ml, that the crystallization is carried out within a pH range from 1 pH unit below the isoelectric point to 1 pH unit above this point, and that the phenol is the monohydroxybenzene and/or cresol.

5. Process according to one or more of claims 1 to 4, characterized in that it is carried out in the presence of a phosphate or citrate buffer.

6. Process according to one or more of claims 1 to 5, characterized in that the prepared crystal suspensions additionally contain at least one compound of the group comprising a) insulin derivatives of the formula I in dissolved and/or amorphous form, b) insulin, c) proinsulin and d) C-peptide, in each case in dissolved, amorphous and/or crystalline form and, where appropriate, additionally an auxiliary having a delaying action.

7. Process for the preparation of crystal suspensions according to one or more of claims 1 to 6 for use as medicine.

8. Process for the preparation of crystal suspensions according to one or more of claims 1 to 6 for use as medicines in the treatment of diabetes mellitus.

**Revendications** (pour les Etats contractants : BE, CH, DE, FR, GB, IT, LI, LU, NL, SE)

1. Procédé de préparation de suspensions de cristaux de forme régulière ayant une répartition de tailles très homogène, en particulier des prismes d'une taille d'environ 10 μm, d'un ou plusieurs dérivés d'insuline de formule I

(Voir formule page suivante)

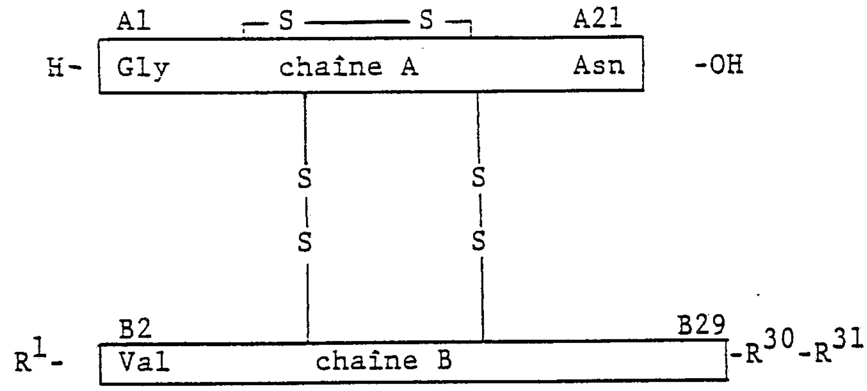

dans laquelle

$R^1$ représente H ou H-Phe

$R^{30}$ représente le radical d'un acide L-aminé neutre pouvant être génétiquement codé et

$R^{31}$ représente un groupe organique physiologiquement acceptable de caractère basique ayant jusqu'à 50 atomes de carbone, à la constitution duquel participent de 0 à 3 α-amino-acides et dont la fonction carboxy en position finale éventuellement présente peut être sous forme de fonction ester, sous forme de fonction amide, sous forme de lactone ou réduite en $CH_2OH$, avec un point isoélectrique entre 5,8 et 8,5,

caractérisé en ce que la cristallisation est conduite en milieu aqueux au voisinage du point isoélectrique du ou des dérivés en présence de glycérine comme agent d'isotonie et d'au moins un phénol.

2. Procédé selon la revendication 1, caractérisé en ce que dans la formule I :

$R^{31}$ représente un radical de formule —$X_nS$, dans lequel n = 0, 1, 2 ou 3

X représente des radicaux identiques ou différents d'acides L-aminés neutres ou basiques que l'on trouve dans la nature, de préférence d'acides L-aminés basiques, en particulier Arg, Lys, His, Hyl ou Orn et/ou des acides D-aminés qui leur correspondent et

S représente OH ou un groupe physiologiquement acceptable bloquant le groupe carboxy qui, si n = 0, porte un radical basique chargé positivement ou protonable ou si n est supérieur à 0, peut porter un tel radical et où l'extrémité C

—X—S peut également représenter le radical d'un acide aminé réductible en l'alcool correspondant ou, si n = 2 ou 3, le radical homosérine lactone.

3. Procédé selon la revendication 1 ou 2, caractérisé par au moins une des caractéristiques selon lesquelles dans la formule I $R^1$ représente H-Phe, $R^{30}$ représente Ala, Thr ou Ser et le radical $R^{31}$ contient des radicaux acides aminés ou des radicaux de leurs dérivés exclusivement sous forme L.

4. Procédé selon une ou plusieurs des revendications 1 à 3, caractérisé par au moins l'une des caractéristiques suivantes : dans le milieu de cristallisation on trouve une quantité de zinc allant jusqu'à 1 % par rapport à la masse d'insuline ; la cristallisation est conduite à des températures de 3 à 27 °C ; la concentration du dérivé d'insuline (I) dans le milieu de cristallisation s'élève à 0,2 à 40 mg/ml ; la cristallisation est conduite dans un intervalle de pH allant d'une unité de pH en-dessous du point isoélectrique jusqu'à une unité de pH au-dessus de ce point ; et le phénol est le monohydroxybenzène et/ou le crésol.

5. Procédé selon une ou plusieurs des revendications 1 à 4, caractérisé en ce qu'on travaille en présence d'un tampon phosphaté ou citraté.

6. Suspension de cristaux d'un ou plusieurs dérivés d'insuline de formule I que l'on peut obtenir par un procédé selon une ou plusieurs des revendications 1 à 5.

7. Suspension de cristaux selon la revendication 6, caractérisée en ce qu'elle contient en outre au moins un composé du groupe a) dérivés d'insuline de formule I sous forme dissoute et/ou amorphe, b) insuline, c) proinsuline et d) C-peptide chacun sous forme dissoute, amorphe ou cristalline et éventuellement en outre un additif à action retard.

8. Suspension de cristaux selon la revendication 6 ou 7, pour utilisation comme médicament.

9. Suspension de cristaux selon l'une des revendications 6 ou 7, pour l'utilisation comme médicament dans le traitement du diabète sucré.

10. Composé de formule I sous forme de cristaux de forme régulière à répartition de tailles largement homogène, en particulier des prismes d'une taille d'environ 10 µm, que l'on peut obtenir par un procédé selon une ou plusieurs des revendications 1 à 5.

**Revendications** (pour l'Etat contractant AT)

1. Procédé de préparation de suspensions de cristaux de forme régulière ayant une répartition de

tailles très homogène, en particulier des prismes d'une taille d'environ 10 μm, d'un ou plusieurs dérivés d'insuline de formule I

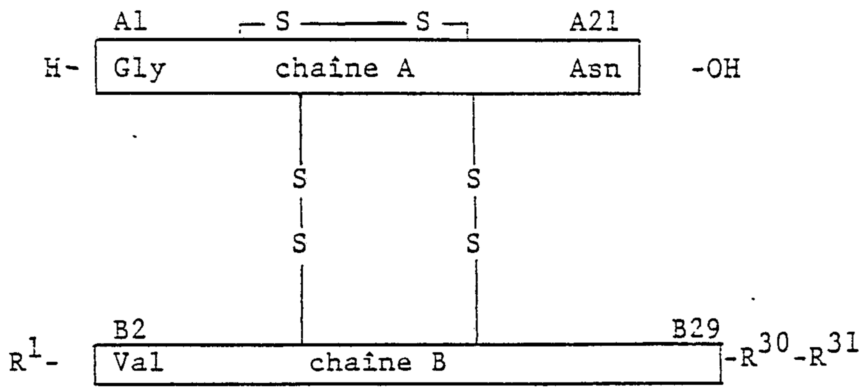

dans laquelle

$R^1$ représente H ou H-Phe

$R^{30}$ représente le radical d'un acide L-aminé neutre pouvant être génétiquement codé et

$R^{31}$ représente un groupe organique physiologiquement acceptable de caractère basique ayant jusqu'à 50 atomes de carbone, à la constitution duquel participent de 0 à 3 α-amino-acides et dont la fonction carboxy en position finale éventuellement présente peut être sous forme de fonction ester, sous forme de fonction amide, sous forme de lactone ou réduite en $CH_2OH$, avec un point isoélectrique entre 5,8 et 8,5,

caractérisé en ce que la cristallisation est conduite en milieu aqueux au voisinage du point isoélectrique du ou des dérivés en présence de glycérine comme agent d'isotonie et d'au moins un phénol.

2. Procédé selon la revendication 1, caractérisé en ce que dans la formule I :

$R^{31}$ représente un radical de formule $—X_nS$, dans lequel n = 0, 1 , 2 ou 3

X représente des radicaux identiques ou différents d'acides L-aminés neutres ou basiques que l'on trouve dans la nature, de préférence d'acides L-aminés basiques, en particulier Arg, Lys, His, Hyl ou Orn et/ou des acides D-aminés qui leur correspondent et

S représente OH ou un groupe physiologiquement acceptable bloquant le groupe carboxy qui, si n = 0, porte un radical basique chargé positivement ou protonable ou si n est supérieur à 0, peut porter un tel radical et où l'extrémité C

—X—S peut également représenter le radical d'un acide aminé réductible en l'alcool correspondant ou, si n = 2 ou 3, le radical homosérinelactone.

3. Procédé selon la revendication 1 ou 2, caractérisé par au moins une des caractéristiques selon lesquelles dans la formule I $R^1$ représente H-Phe, $R^{30}$ représente Ala, Thr ou Ser et le radical $R^{31}$ contient des radicaux acides aminés ou des radicaux de leurs dérivés exclusivement sous forme L.

4. Procédé selon une ou plusieurs des revendications 1 à 3, caractérisé par au moins l'une des caractéristiques suivantes : dans le milieu de cristallisation on trouve une quantité de zinc allant jusqu'à 1 % par rapport à la masse d'insuline ; la cristallisation est conduite à des températures de 3 à 27 °C ; la concentration du dérivé d'insuline (I) dans le milieu de cristallisation s'élève à 0,2 à 40 mg/ml ; la cristallisation est conduite dans un intervalle de pH allant d'une unité de pH en-dessous du point isoélectrique jusqu'à une unité de pH au-dessus de ce point et le phénol est le monohydroxybenzène et/ou le crésol.

5. Procédé selon une ou plusieurs des revendications 1 à 4, caractérisé en ce qu'on travaille en présence d'un tampon phosphaté ou citraté.

6. Procédé selon une ou plusieurs des revendications 1 à 5, caractérisé en ce que la suspension de cristaux préparés contient en outre au moins un composé du groupe a) dérivés d'insuline de formule I sous forme dissoute et/ou amorphe, b) insuline, c) proinsuline et d) C-peptide chacun sous forme dissoute, amorphe et/ou cristalline et éventuellement en outre un additif à action retard.

7. Procédé de préparation de suspensions de cristaux selon une ou plusieurs des revendications 1 à 6, pour utilisation comme médicament.

8. Procédé de préparation de suspensions de cristaux selon une ou plusieurs des revendications 1 à 6, pour utilisation comme médicament dans le traitement du diabète sucré.

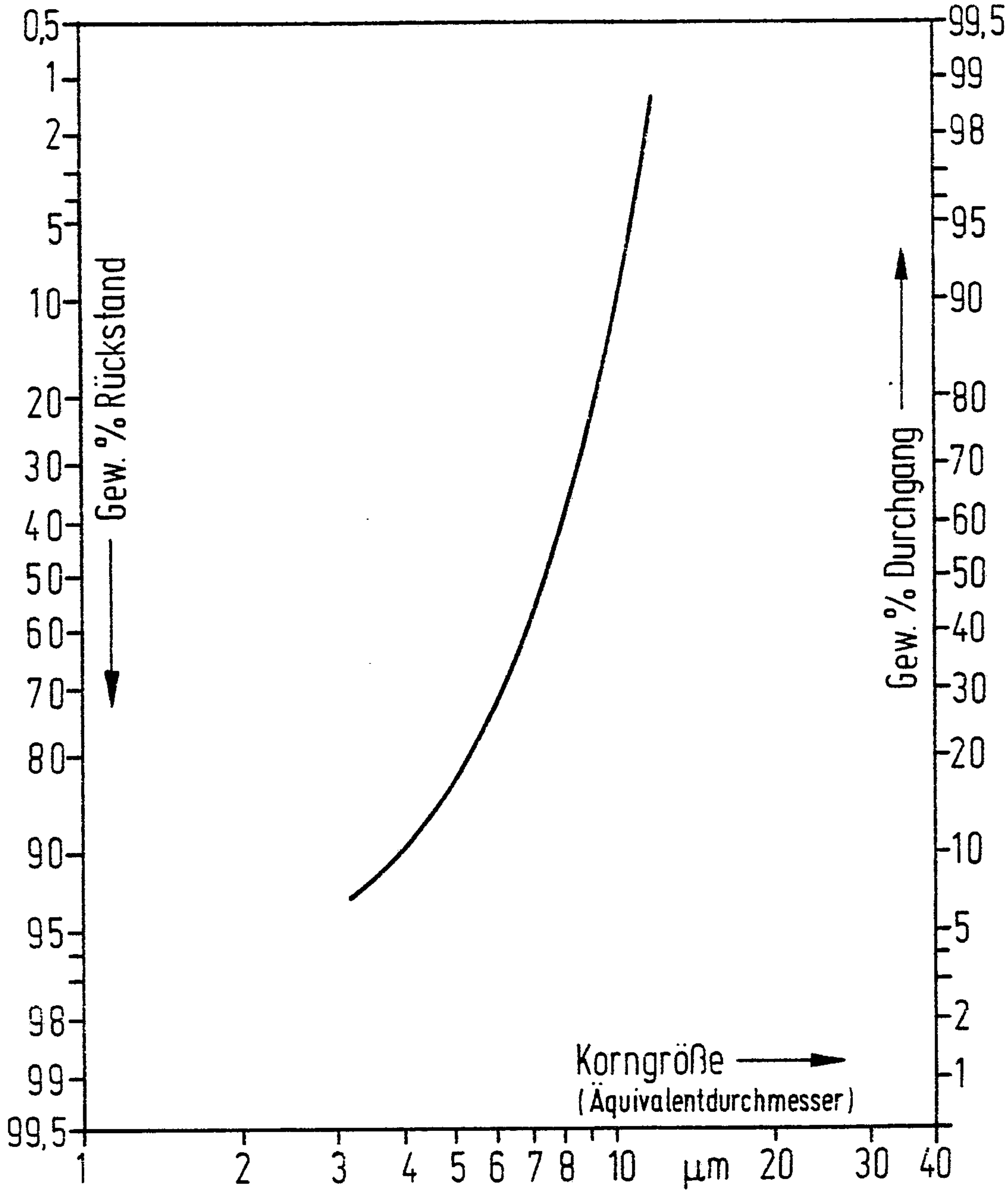
0,5
1
2
5
10
20
30
40
50
60
70
80
90
95
98
99
99,5
Gew. % Rückstand
99,5
99
98
95
90
80
70
60
50
40
30
20
10
5
2
1
Gew. % Durchgang
Korngröße
(Äquivalentdurchmesser)
1
2
3
4
5
6
7
8
10
µm
20
30
40